# EUROPEAN PATENT APPLICATION

(11) **EP 4 785 887 A1**
(43) Date of publication of application: **05.08.2026**
(21) Application number: 26152009.2
(22) Date of filing: 15.01.2026
(51) Int. Cl.: A61B 18/02

(54) **MULTI-FILAR COIL HEATERS FOR CRYOABLATION PROBES**

(30) Priority: 31.01.2025 US 202519042565
(71) Applicant: Varian Medical Systems, Inc., Palo Alto, CA 94304 (US)
(72) Inventor: MAY, Justin, Austin, 78717 (US)
(74) Representative: Mathisen & Macara LLP

(57) **Abstract**

A cryoprobe (300) includes a needle (304) defining an internal cavity, a cryogen conduit (308) positioned in the internal cavity, and a multi-filar heater (314) positioned radially outward of the cryogen conduit in the internal cavity configured to heat the needle to a predetermined temperature.

## Description

### FIELD

The present disclosure relates to multi-filar heaters that may be used in cryoablation probes.

### BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through a probe in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

Traditional or existing cryoprobes and related structures may use a second gas or fluid that is passed through the cryogen flow path to warm the cryoprobe. The cryoprobe may be warmed, for example, using helium or another gas. The second gas may warm the cryoprobe following a freezing cycle in order to permit the cryoprobe to be removed from the ice that forms during the freezing cycle. The use of a second gas or fluid adds complexity and cost to the cryoablation system. There exists a need, therefore, for improved cryoablation systems and probes that may allow for warming or heating to be performed without adding significant cost and/or complexity to the cryoablation system.

### SUMMARY

This section provides a general summary of the disclosure, and is not a comprehensive disclosure of its full scope or all of its features.

In various embodiments of the present disclosure, a cryoprobe is provided. The cryoprobe may include a resistive heater that is made of a multi-filar wire. The multi-filar wire may include a structural member and a plurality of heating members. The structural member may be used to add rigidity and/or shape to the resistive heater. The plurality of heating members may be used to heat the cryoprobe at or near the resistive heater. In some examples, the cryoprobe may include an adjustable insulating sleeve that may be configured to move axially or longitudinally in the needle of the cryoprobe. Movement of the insulating sleeve allows a user to adjust a size of the iceball that is produced by the cryoprobe. The resistive heater may be adjustable and configured to adjust in longitudinal length with the adjustable vacuum sleeve.

In some embodiments of the present disclosure, the cryoprobe may include a needle defining an internal cavity, a cryogen conduit positioned in the internal cavity, and a multi-filar heater positioned radially outward of the cryogen conduit in the internal cavity configured to heat the needle to a predetermined temperature. The needle is suitably an elongate member, for example a cylindrical member having a pointed tip. The needle may have an outer diameter between an outer diameter between about 1 mm to about 4 mm. The needle may define the outer surface of cryoprobe. The cryogen conduit may be positioned centrally in the needle. In another example, the cryogen conduit may be offset from the axis of the needle, while still positioned in the cavity defined by the needle. The heater may be positioned in or on the needle of the cryoprobe. The heater may be located radially inward of the needle and/or inside the inner cavity of the needle. The heater may be located at a position along the needle between a tip of the needle and the insulating sleeve. The heater may be configured to raise the temperature of the needle to a temperature that is greater than about 80 degrees Celsius. The cryoprobe may further comprise a handle configured with a first/proximate portion and a second/distal portion, where the first portion is substantially aligned with the cryogen line and the second portion is offset relative to the first portion. The first portion may be offset relative to the second portion at any angle ergonomically suitable for use. In another embodiment the cryoprobe is substantially linear with the handle substantially aligned in the same direction as the needle.

In one aspect, the cryoprobe may include an adjustable insulating sleeve positioned in the inner cavity of the needle, radially outward of the cryogen conduit. The insulating sleeve may be a cylindrical length of insulation positioned in the needle. Suitably, the insulating sleeve is configured to prevent or reduce the transfer of thermal energy between the cryogen inside the needle and the tissue that is positioned externally to the needle during use.

In another aspect, the adjustable insulating sleeve is configured to move axially in the internal cavity of the needle. The cryoprobe may also include an adjustor connected to the insulating sleeve. The adjustor may be configured to move the insulating sleeve inside the needle. The adjustor may be a knob, button, pin, locking screw, or other adjustment control that may allow the user to move the insulating sleeve by moving the adjustor, which may be located in the handle. The adjustor may move in a slot or between various detents to allow the insulating sleeve to be positioned at one or more predetermined axial positions relative to the tip of the cryoprobe. In other examples, the adjustor may allow the position of the insulating sleeve to be continuously variable along the needle. The adjustor may be fixed once the insulating sleeve is located at a desired position.

In another aspect, the insulating sleeve may be a hollow cylindrical member. In an aspect, the adjustable insulating sleeve may be a vacuum sleeve. The insulating sleeve may be formed by an inner cylindrical wall and an outer cylindrical wall. The two tubes of material may be joined fixing the inner cylindrical wall to the outer cylindrical wall to form an inner cavity that may be drawn to a vacuum before the ends of the outer wall are closed. The inner wall (or tube) may have length that is greater than a length of the outer wall, such that a portion of the inner wall extends away from the outer wall toward the tip. The heater may be coiled around the portion of the inner wall 616 that projects out from the outer wall.

In another aspect, the multi-filar heater may include a coil of multi-filar wire. The multi-filar wire may include multiple members or wires that may extend alongside each other under an outer insulating cover.

In another aspect, the insulating sleeve or vacuum sleeve may be movable axially along a length of the needle. The insulating sleeve may be adjusted to be between 0.5-33 mm from the tip of the needle. In an aspect, the heater may be connected to the adjustable insulating sleeve at one of more locations, and configured to move with the insulating sleeve in the needle. For example, the second end of the heater may be connected to a distal end of the insulating sleeve, being the end of the insulating sleeve closest to the tip of the needle. The heater may be in contact with the inner surface of the needle. Alternatively, the diameter of the heater may be less than the inner diameter of the needle. In an aspect, the multi filar heater may be connected to the adjustable insulating sleeve and may extend or contract with a movement of the adjustable insulating sleeve. In an aspect, the heater may be configured to elongate or contract when the insulating sleeve is moved in an axial direction in the needle, for example, the heater may elongate similarly to a spring when the insulating sleeve is moved in a direction away from the tip and contract when the insulating sleeve is moved in a direction toward the tip. In another aspect, the heater has a fixed or constant length. In this embodiment, the heater does not expand or contract when moving with the insulating sleeve. The heater may have a length of about 0.3-0.4 mm when contracted or at its fixed length. The heater may extend to about 30-35 mm.

In another aspect, a first end of the multi-filar heater may be connected to the cryogen conduit and a second end of the heater may be connected to the insulating sleeve. In another aspect, the heater is positioned at or around the cryogen conduit, such that the heater may be connected to the cryogen conduit to remain in a fixed position in the needle. In this aspect, the heater may be coiled or wrapped around the cryogen conduit. The outer diameter of the heater is suitably sized so that the insulating sleeve may slide over the heater when the insulating sleeve is adjusted to a desired axial position in the needle.

In another aspect, the multi-filar heater may include a multi-filar wire comprising a structural wire and a plurality of heater wires.

In another aspect, an outer diameter of the structural wire may be greater than an outer diameter of each heater wire of the plurality of heater wires. In another aspect the structural wire and the plurality of heater wires may have the same or a similar outer diameter. The structural wire may be a length of material such as a metal, alloy, or plastic material, in some examples, steel, aluminum, or other material may be used. The structural wire may be the same size or have the same outer diameter as the heater wires. In other examples, the structural wire may be larger or smaller or have a different outer diameter than the heater wires. The structural member may allow the multi-filar wire to be shaped in a desired configuration such as a coil, helix, or other shape. In some examples, the structural member may be deformable and may return to a desired shape. The structural wire may be formed of a spring metal.

In another aspect, the multi-filar wire may include a cover. The cover may surround and/or cover the structural member wire and the heater wires. The cover may be made of an insulating material. The cover may be a heat shrink or polymer tubing material. In some examples, the cover may be a thin layer of insulating material that is applied to the outer surface of the structural member and the heating wires. The cover may join the wires or members to each other. In some examples, a polyimide material may be used for the cover. Other materials may be used in other examples.

In another aspect, the multi-filar wire may have a helical shape.

In another aspect, the plurality of heater wires may be deposited on an external surface of the structural wire. For example, the heater wires may be deposited by printing a conductive resistive ink on the structural wire.

In another aspect, each heater wire of the plurality of heater wires may include an external insulating layer.

In another aspect, the structural wire may include a nickel and titanium alloy or nitinol configured to move to predetermined shape at a predetermined temperature.

In another aspect, the plurality of heater wires may include a first heater wire and a second heater wire. The first heater wire and the second heater wire may have similar properties, sizes and shapes. The first heater member and the second heater member may include an insulating cover around the conductive portion of the member to electrically insulate the conductive portions from each other. The first heater wire and the second heater wire may be joined together at or near the distal end of the needle. The first heater wire and the second heater wiew may form a heating circuit that heats when a current is passed through the heater wires. The first heater wire and the second heater wire along with the structural wire are aligned or positioned next to each other in a linear relationship. In other examples, the first heater wire, the second heater wire and the structural wire may be oriented relative to each other in other configurations in other examples.

In another aspect, each heater wire of the plurality of heater wires may be positioned adjacent to each other.

In another aspect, each heater wire of the plurality of heater wires may be positioned around a circumference of the structural wire.

In another aspect, the multi-filar wire may also include sensor wires that may transmit signals regarding operating conditions of the cryoprobe such as temperature or other parameters. In an aspect of the invention, the multi-filar heater may include at least one temperature sensing wire.

In some embodiments of the present disclosure, a cryoprobe may include a needle defining an internal cavity, a cryogen conduit positioned in the internal cavity, and a multi-filar heater positioned radially outward of the cryogen conduit in the internal cavity configured to heat the needle to a predetermined temperature. The multi-filar heater may be formed of at least one structural wire and a plurality of heater wires wherein at least two of the plurality of heater wires are connected at or near a distal end of the internal cavity to form a heating circuit. In another aspect, the present disclosure provides a cryoablation apparatus comprising a cryo-controller, a cryogen delivery apparatus and a cryoprobe according one or more embedment of the present disclosure. The cryoablation apparatus may comprise a cryoablation console, including a cryo-controller, a cryogen delivery apparatus and a cryogen source. The cryo-controller may include a computing device or other controller that can be used to control delivery of a cryogen from the cryogen source to the cryoprobe using the cryogen delivery apparatus. The cryogen may comprise any suitable cryogen, such as argon or nitrogen. The cryogen source is suitably connected to the cryoprobe by a cryogen line, comprising a flexible tube or similar conduit. The cryogen line may comprise multiple lumens to allow cryogen to flow in a supply direction to the cryoprobe and separately in a return direction away from the cryoprobe.

In one aspect, the multi-filar heater may have a helical shape. The helical shaped multi-filar heater may contact an inner surface of the needle.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an illustration of an example cryoablation apparatus in accordance with some embodiments of the present disclosure.
FIG. 2 is a side sectional view of an example cryoprobe in accordance with some embodiments of the present disclosure.
FIG. 3 is an isometric view of an example cryoprobe in which a portion of needle is transparent to show an extendible heater in accordance with some embodiments of the present disclosure.
FIG. 4 is an isometric view of the cryoprobe of FIG. 3 shown with the insulating sleeve and heater in an intermediate position.
FIG. 5 is an isometric view of the cryoprobe of FIG. 3 shown with the insulating sleeve and heater in a contracted position.
FIG. 6 is an isometric view of another example cryoprobe shown with a portion of the needle transparent to show the heater and internal elements in accordance with some embodiments of the present disclosure.
FIG. 7 is an isometric view of the cryoprobe of FIG. 6 in which the insulating sleeve and heater are located in a position closer to the tip of the cryoprobe.
FIG. 8 is an illustration of another example cryoprobe showing a heater coiled around a cryogen conduit in accordance with some embodiments of the present disclosure.
FIG. 9 is a cross-sectional view of an example multi-filar wire that is used to form a resistive heater in some embodiments of the present disclosure.
FIG. 10 is a cross-sectional view of another example multi-filar wire that is used to form a resistive heater in some embodiments of the present disclosure.
FIG. 11 is a cross-sectional view of another example multi-filar wire that is used to form a resistive heater in some embodiments of the present disclosure.
FIG. 12 is a cross-sectional view of an example wire with leads deposited on a structural wire that is used to form a resistive heater in some embodiments of the present disclosure.
FIG. 13 is a cross-sectional view of another example multi-filar wire that is used to form a resistive heater in some embodiments of the present disclosure.
FIG. 14 is a cross-sectional view of another example multi-filar wire that is used to form a resistive heater in some embodiments of the present disclosure.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In various embodiments of the present disclosure, a cryoprobe is provided that may include a heater. The heater may be configured to heat the needle of the cryoprobe to an elevated temperature. The cryoprobe may include an insulated sleeve, such as a vacuum sleeve. The insulated sleeve may be adjustable or movable relative to a distal end of the needle. The insulated sleeve may be adjusted to control a size of an iceball that is created during a freezing cycle of the cryoprobe. When the insulated sleeve is adjusted to a position closer to a distal end of the needle, the size of the iceball is decreased. When the insulated sleeve is adjusted to a position further away from the distal end of the needle, the size of the iceball is greater. The iceball may be controlled so as to create an iceball that corresponds to a size of the target tumor (e.g., tumor) that may be destroyed during a cryoablation treatment.

Existing cryoablation systems may use a second gas or fluid (e.g., helium) that is passed through the cryogen flow path to warm or heat the needle of the cryoprobe. Such systems suffer from various drawbacks. In such systems, the use of a second gas increases the complexity of the systems because a second cryogen tank or source is required as well as conduits, valves, pumps, or the like may be necessary to control the flow of the second gas through the cryogen flow path. In addition, secondary gases (e.g., Helium) may be consumed during heating operations and the use of the secondary gas adds cost to the treatment and to cost to overall cryoablation system.

The cryoablation apparatuses and cryoprobes of the present disclosure may include a heater positioned in or on the needle of the cryoprobe that do not utilize a secondary gas. Instead, the heaters may use conduction, convection, or other heating methods to transfer heat from the heater to the needle. In some embodiments, the heaters may be a resistive heater that may heat when a current is passed through the heater. Existing cryoablation systems with movable or adjustable insulting sleeves do not include resistive heaters or other localized heaters.

In some embodiments, the resistive heater may be formed from a multi-filar wire. Such a multi-filar wire may include a plurality of longitudinal wires or other members. In some examples, the multi-filar wire includes a structural member or wire that can provide rigidity to the heater and may be used to form a shape of the heater. The multi-filar wire may also include heater wires that may heat when current is passed through them and/or sensor wires that may transmit signals regarding operating conditions of the cryoprobe such as temperature or other parameters.

The cryoprobes and cryoablation apparatuses of the present disclosure are improvements over known apparatuses and methods. The cryoprobes of the present disclosure may allow heating to efficiently performed. The cryoprobes of the present disclosure may also allow heating procedures to be performed that may not be possible using existing cryoprobes with adjustable insulating sleeves. For example, the cryoprobes of the present disclosure may allow for cautery and track ablation procedures to be performed that are not possible using a cryoprobe that uses a secondary gas for warming. The cryoprobes of the present disclosure may achieve temperatures that are greater than about 80 degrees Celsius. Such a temperature allows a cautery procedure or a track ablation procedure to be performed in which the tissue surrounding the needle is heated to a temperature that destroys abnormal tissue and/or may prevent or reduce bleeding during needle re-positioning or withdrawal.

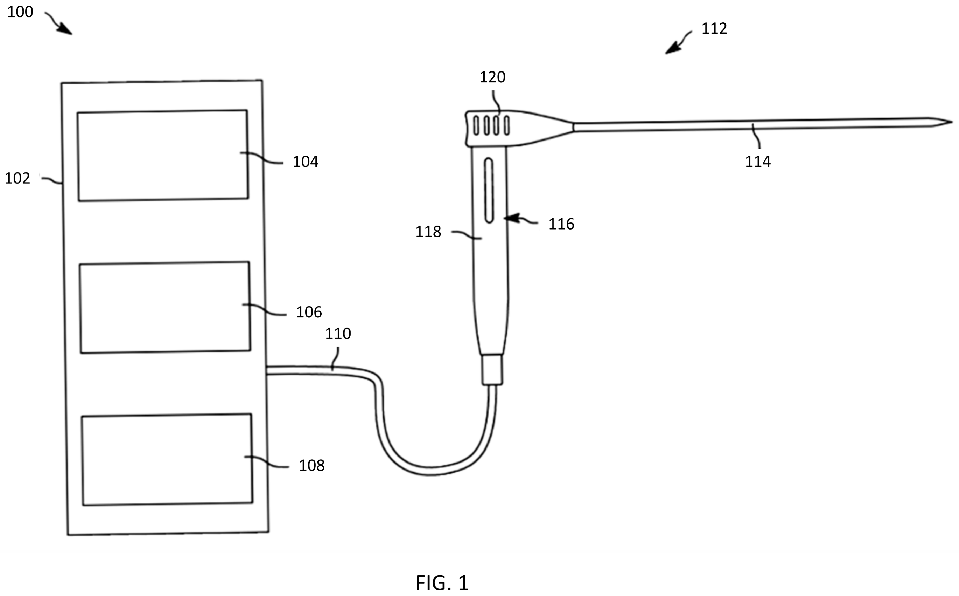

Referring now to FIG. 1, an example cryoablation apparatus 100 is shown. The cryoablation apparatus 100 may include a cryoablation console 102, and a cryoprobe 112. The cryoablation console 102 may include a cryo-controller 104, a cryogen delivery apparatus 106 and a cryogen source 108. The cryo-controller 104 may include a computing device or other controller that can be used to control delivery of a cryogen (e.g., Argon, Nitrogen, or the like) from the cryogen source 108 to the cryoprobe 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe 112. Upon the initiation of a freezing cycle, the cryo-controller 104 may cause the cryogen to be moved through a cryogen flow path that includes a cryogen supply line from the cryogen source through the cryogen line 110 to the cryoprobe 112. The cryogen may then flow back to the cryogen source via a cryogen return line from the cryoprobe 112 back to the cryogen source 108.

The cryogen line 110 may be a flexible tube or other conduit that may include multiple lumens to allow the cryogen to flow in a supply direction to the cryoprobe 112 and separately in a return direction away from the cryoprobe 112. The cryogen line 110 may be of sufficient length to allow the console 102 to be positioned near the patient in a treatment room and to allow the patient to be moved into and out of an imaging device. In some examples, the cryogen line may be at least about 3.75 meters (12 feet) in length. In other examples, the cryogen line 110 may have other lengths.

The cryogen line 110 fluidly couples the cryogen source 108 to the cryoprobe 112. The cryoprobe 112 may include a needle 114. The needle 114 may be a pointed cylindrical tool or other elongated member that is configured to be inserted into patient tissue and be positioned at or near the target tissue during treatment. The needle 114 may be configured as a pointed tool having an outer diameter in a range of about 1 mm to about 4 mm. The cryoprobe 112 may also include a handle 116. The handle 116 may be configured with a first (or proximate) portion 118 and a second (or distal) portion 120. The first portion 118 may be substantially aligned with the cryogen line 110 and the second portion 120 may offset at an angle relative to the first portion 118. The offset angle between the first portion 118 and the second portion 120 may be about 90 degrees to define a right angle handle. In other example, the first portion 118 and the second portion 120 may be offset at different angles. In still other examples, the cryoprobe 112 may be substantially linear and the handle 116 may be aligned in a same direction as the needle 114.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 112. This may allow an operator to touch or otherwise manipulate the cryoprobe 112 during treatment.

The cryoprobe 112 may also include an insulated sleeve positioned in the needle 114. The insulated sleeve may be a cylindrical length of insulation that may prevent or reduce the transfer of thermal energy between the cryogen inside the needle 114 and the tissue that is positioned externally to the needle 114 during a treatment. The insulated sleeve may be a hollow cylindrical member that may be positioned radially inward of the needle 114 and radially outward of an inner conduit in the needle that supplies cryogen toward the tip of the needle. The insulated sleeve may be a vacuum sleeve in which the hollow space inside the slide is manufactured to be at vacuum so as to prevent or reduce the transfer of thermal energy. In some examples, the insulated sleeve or vacuum sleeve may be movable axially along a length of the needle 114. In this manner, the size of the iceball that is created during a freezing may be adjusted to have a desired size. An example movable or adjustable insulating sleeve is described further in U.S. Patent No. 11,877,781 to Varian Medical Systems, Inc., the contents of which are incorporated herein by reference.

While not shown, more than one cryoprobe 112 may be coupled to the console 102. Multiple cryoprobes 112 may be used during a single cryoablation treatment in combination. The console 102 may be configured to deliver cryogen to each of the multiple cryoprobes 112. The cryoprobes 112 may be similar to reach other or may be different to produce iceballs of different sizes and shapes so as to freeze and destroy the target tissue.

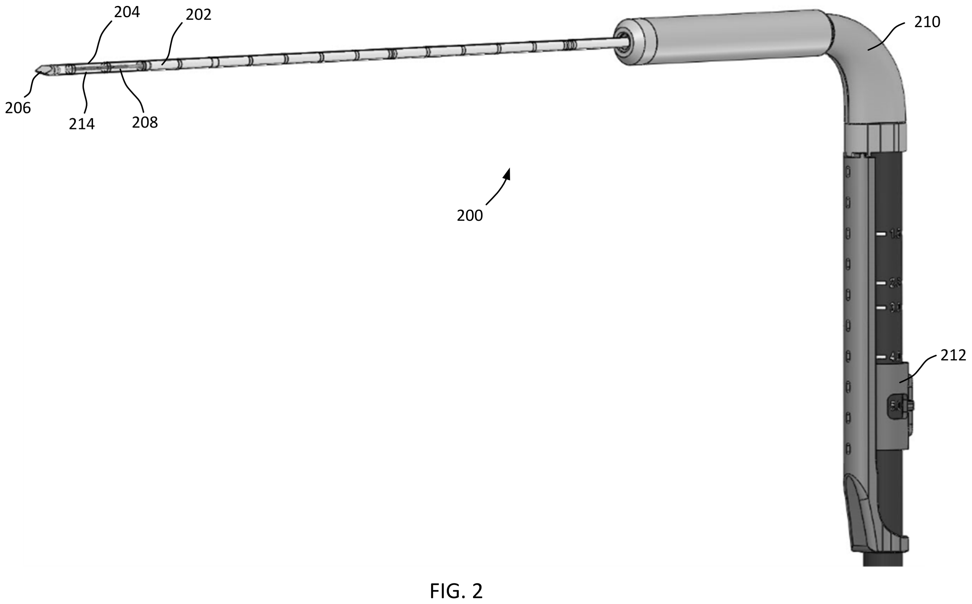

Referring now to FIG. 2, an example cryoprobe 200 is shown. In this example, the cryoprobe 200 includes a movable or adjustable insulating sleeve 202 as previously described. The cryoprobe 200 includes a needle 204, a tip 206, cryogen conduit 208, and handle 210. The needle 204 may extend from the handle 210 and may be the outer member of the cryoprobe 200 that is positioned into the patient at or near the target tissue. Thus, the outer surface of the needle 204 may be positioned in the tissue of the patient. The cryogen conduit 208 may be positioned inside the needle 204. In some examples, the cryogen conduit 208 may be positioned centrally in the needle 204. In other examples, the cryogen conduit 208 may be offset from the axis of the needle 204 while still positioned in the cavity defined by the needle 204.

During a freezing cycle, cryogen (e.g., Argon, Nitrogen) may be passed through the cryogen conduit 208 toward the tip 206 at a low temperature. The flow of cryogen exits the cryogen conduit 208 and then flows away from the tip 206 toward the handle 210. The return path of the cryogen may be between an outer surface of the cryogen conduit 208 and an inner surface of the needle 204. As the cryogen flows down the needle toward the handle 210, the cryogen may flow between the cryogen conduit 208 and the inner surface of the insulating sleeve 202. The insulating sleeve 202 may insulate the needle 204 from the cryogen at a portion of the longitudinal length of the needle. At the portions of the needle 204 that include the insulating sleeve 202, thermal energy transfer between the tissue surrounding the needle 204 and the needle 204 is limited.

The cryoprobe 200 may also include an adjustor 212 that may be connected to the insulating sleeve 202. The adjustor 212 may be used to move the insulating sleeve 202 inside the needle 204. The adjustor 212 may be a knob, button, pin, locking screw, or other adjustment control that may allow the user to move the insulating sleeve 202 by moving the adjustor 212 in the handle 210. The adjustor 212 may move in a slot or between various detents to allow the insulating sleeve 202 to be positioned at one or more predetermined axial positions relative to the tip 206 of the cryoprobe 200. In other examples, the adjustor 212 may allow the insulating sleeve 202 to be continuously variable along the needle 204. The adjustor 212 may also be fixed once a desired location of the insulating sleeve 202 is located at a desired position.

The cryoprobe 200 may also include heater 214. The heater 214 may be located at a position along the needle 204 between the tip 206 and the insulating sleeve 202. In the example shown, the heater 214 may be located radially inward of the needle 204 and/or inside the inner cavity of the needle 204. In other examples and as further described below, the heater 214 may be located at other locations. The heater 214 may be coupled to a power source that may be located in a cryoablation console or other cryoablation apparatus. The power source may supply a power signal to the heater 214 to cause the heater to warm or heat the needle 204. In some examples, the heater 214 may be configured to raise a temperature of the needle 204 to perform a thaw procedure to allow the needle 204 to be extracted from an iceball following a freezing cycle. In other examples, the heater 214 may be configured to raise a temperature of the needle 204 to perform a thaw procedure and may also allow the needle 204 to perform a cautery or track ablation procedure. In such examples, the heater 214 may be configured to raise the temperature of the needle 204 to a temperature that is greater than about 80 degrees Celsius.

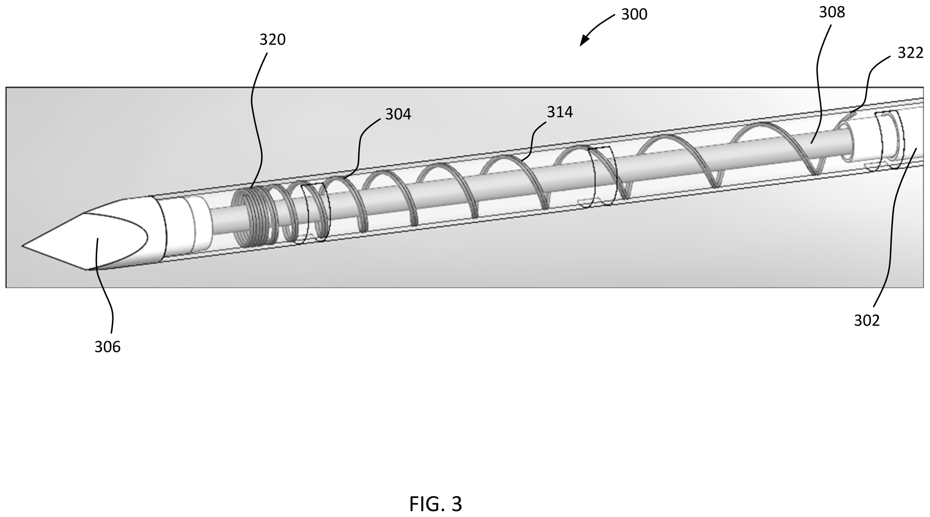

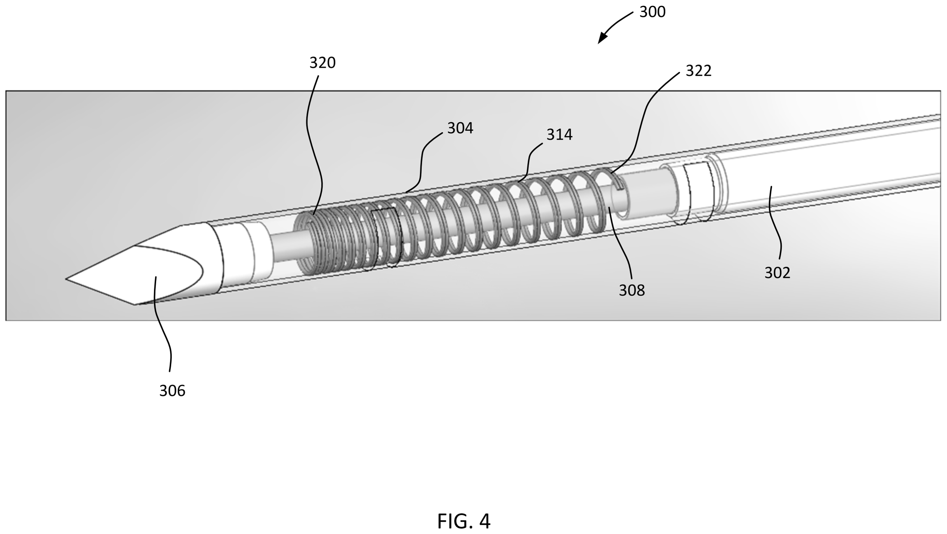

Referring now to FIGs. 3 to 5, another example cryoprobe 300 is shown. The cryoprobe 300 may be similar to the cryoprobe 200 previously described. The cryoprobe 300 may include an insulating sleeve 302, needle 304, tip 306, cryogen conduit 308, and a heater 314. The needle 304 and the tip 306 may be similar to the needle 204 and tip 206 previously described. The needle 304, in FIG. 3, is shown partially transparent to illustrate the internal elements and configuration of the cryoprobe 300. The needle 304 is a continuous tube of material with the tip 306 positioned at one end to define the internal cavity. The cryoprobe 300 may operate as previously described to perform a freezing cycle when a cryogen flows through a cryogen flow path. The cryogen may flow toward the tip 306 through the cryogen conduit 308 and then back away from the tip 306 in the space between the outside of the cryogen conduit 308 and the inner surface of the needle 304.

To perform a heating procedure, such as a thaw, cautery, or track ablation procedure, the heater 314 may be activated. The heater 314, in this example, may be a resistive heater formed of a resistive wire that is coupled to a power source. The heater 314, in some examples, may be formed of a multi-filar wire. The multi-filar wire may include multiple members or wires that may extend along side each other under an outer insulating cover. The multi-filar wire, as further described below, may include a structural member and plurality of heater members. The structural member may provide rigidity and allow the multi-filar heater to be shaped into a helical, coil or other configuration. The heater members may be made of a suitable resistive material that may heat when current is passed through the member. The heater members may follow a shape of the structural member. In various examples, the heater members may form a heating circuit in the heater to allow current to be supplied to the multi-filar heater.

The heater 314 may be formed in a helical coil configuration. A first end 320 of the heater 314 may be connected at or near the tip 306 of the cryoprobe 300. A second end 322 of the heater 314 may be connected to the distal end of the insulating sleeve 302. The distal end of the insulating sleeve 302 may be the end of the insulating sleeve 302 located closest to the tip 306 of the cryoprobe 300. The first end 320 and the second end 322 of the heater 314 may be connecting using suitable joining techniques such as by soldering, brazing, crimping, or connecting using an adhesive. An epoxy, for example, may be used to connect one or both ends of the heater 314 in the cryoprobe 300.

The heater 314 may be connected to be positioned inside the inner cavity of the needle 304. The heater 314 may be in contact with the inner surface of the needle 304 to convey thermal energy to the needle 304 via conduction. In other examples, the diameter of the heater 314 may be smaller than the inner diameter of the needle 304. In such instances, thermal energy may be transferred to the needle 304 via convection. In still other instances, a combination of conduction and convection may be used to transfer thermal energy to the needle 304.

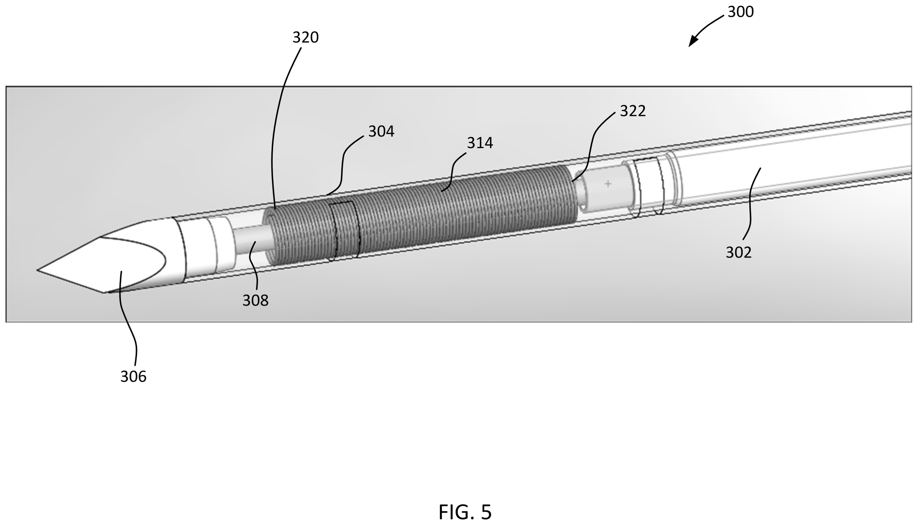

The heater 314 may be configured to elongate or contract when the insulating sleeve 302 is moved in an axial direction in the needle 304. The heater 314 may elongate similarly to a spring when the insulating sleeve 302 is moved in a direction away from the tip 306. Conversely, the heater 314 may contract when the insulating sleeve 302 is moved in a direction toward the tip 306. The heater 314 may operate to perform the heating procedures previously described (i.e., thaw, cautery, and/or track ablation) in any of its positions at any length. The heater 314 may operate in an elongated position in which individual coils of the heater 314 may be spaced apart from each other (FIG. 3). In this position, the insulating sleeve 302 may be positioned in an axial location that is furthest from the tip 306. The heater 314 may also operate in a contracted position in which individual coils of the heater 314 are positioned adjacent or near to each other (FIG. 5). In this position, the insulating sleeve 302 may have been moved from the position shown in FIG. 3 to a position closer to the tip 306. The heater 314 may also operate in an intermediate position in which the individual coils of the heater 314 are spaced apart from each at a spacing that is less than the spacing of the elongated position (FIG. 3) but greater than the spacing of the contracted position (FIG. 5). While only one intermediate position is illustrated, the heater 314 and cryoprobe 300 may be operable in multiple intermediate positions between the elongated position and the contracted position.

With the configuration shown in FIGs. 3 to 5 and described above, the heater 314 may elongate or contract with the movement of the insulating sleeve 302. The heater 314 may distribute thermal energy to the needle 304 in the region between the tip 306 and the insulating sleeve 302. It should be appreciated that the multi-filar heater of the present disclosure may be used in other cryoprobes that may not include an adjustable vacuum sleeve. In such examples, the multi-filar heater may be positioned similarly as that described above but may not be connected to an adjustable sleeve. The multi-filar heaters of the present disclosure can be positioned in needle 304 and may be stationary.

The cryoablation apparatus and/or the cryo-controller that may be coupled to the cryoprobe 300 may control a power signal that is delivered to the heater 314. The power signal may be adjusted as needed to achieve the desired temperature for the heating procedure. The heater 314 may be configured to provide a temperature measurement to the cryo-controller. The heater 314 may be made of a suitable wire material that may have a known relationship between its resistance and temperature so that a temperature of the heater 314 may be determined by the cryo-controller. This information may be obtained and used by the cryo-controller to adjust the power signal delivered to the heater 314 to achieve a predetermined or desired temperature. In some examples, the heater 314 may be formed of an Alloy 120 (Balco) material that may include about 70% Nickel and about 30% Iron to perform the functions described above. In other examples, other materials may be used. In still other examples, the cryoprobe 300 may include one or more sensors to provide temperature or other information regarding a performance of the cryoprobe 300.

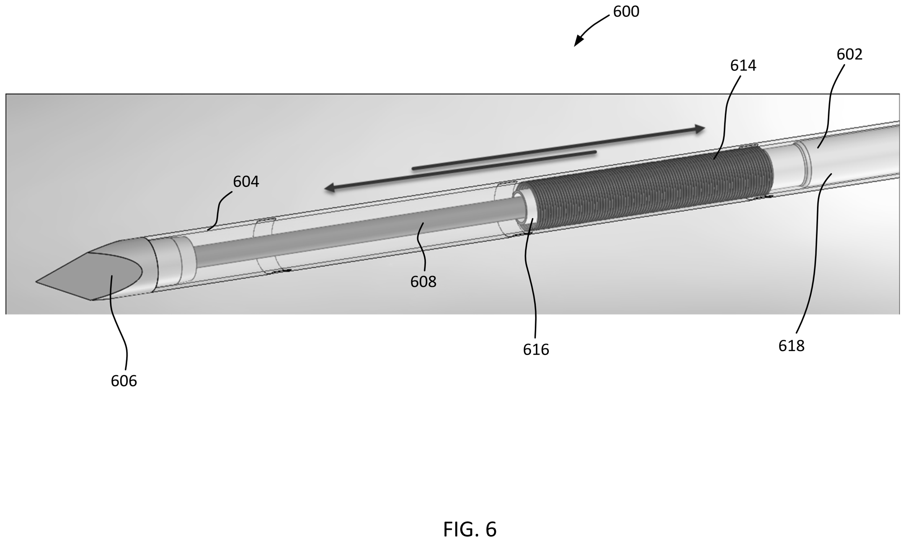

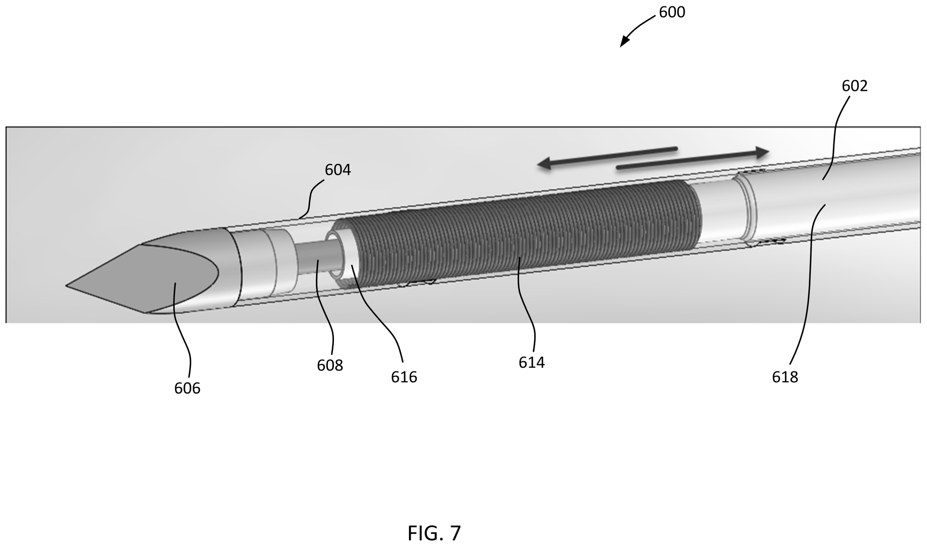

Referring now to FIGs. 6 and 7, another example cryoprobe 600 is shown. In this example, the cryoprobe 600 may be similar and operate similarly to the cryoprobe 200 previously described. As shown, the cryoprobe 600 may include an insulating sleeve 602, a needle 604, a tip 606, a cryogen conduit 608, and a heater 614. As discussed above, the insulating sleeve 602 may be adjustable or movable relative to a tip 606 of the cryoprobe 600. The heater 614, in this example, may be similar to the heater 314 previously described in that the heater 614 may be formed as a coil of resistive wire, such as a multi-filar wire, and may be made of similar materials. The heater 614, in this example, may have a fixed or constant length rather than operating in an expanded and/or contacted state. The heater 614 may be formed by coiling a length of resistive wire and positioning the coil around or radially outward of a portion of the insulating sleeve 602. The heater 614 may be connected to the insulating sleeve 602 at one or more locations and the heater 614 may move with the insulating sleeve 602 in the needle 604.

The insulating sleeve 602 may be formed by an inner cylindrical wall 616 and an outer cylindrical wall 618. The two tubes of material may be joined by brazing, soldering or otherwise fixing the inner cylindrical wall 616 to the outer cylindrical wall 618 to form an inner cavity that may be drawn to a vacuum before the ends of the outer wall 618 are closed. The inner wall 616 (or tube) may have length that is greater than a length of the outer wall 618. In this manner, a portion of the inner wall 616 extends away from the outer wall 618 toward the tip 606. The heater 614 may be coiled around the portion of the inner wall 616 that projects out from the outer wall 618. Since the diameter of the inner wall 616 is less than the diameter of the outer wall 618, the heater 614 may reside in a position radially outward of the inner wall 618 and radially inward of the inner surface of the needle 604.

The heater 614 may have various suitable lengths but may be sized so as to achieve necessary heating temperatures during heating procedures while also allowing the cryoprobe 600 to be adjusted to form suitably sized iceballs during freezing cycles. In some examples, the insulating sleeve 602 may be adjusted to be spaced about 7.6 mm (0.3 inches) to about 33 mm (1.3 inches) from the tip 606. The heater 614 may have a length of about 7.6 mm (0.3 inches) so that it may be used in both the minimum and maximum positions of the insulating sleeve 602. The heater 614 may be used when the cryoprobe 600 is configured to produce a maximum sized iceball in which the insulating sleeve 602 may be located at a position furthest from the tip 606 (FIG. 6). The heater 614 may also be used when the cryoprobe 600 is configured to produce a minimum-sized iceball in which the insulating sleeve 602 is located at a position closest to the tip 606 (FIG. 7).

As discussed above, the heater 614 may be coupled to a power source and energized to perform heating procedures. The heater 614 may be configured to achieve suitable temperatures to perform thaw, cautery, and track ablation procedures in all configurations of the cryoprobe 600 and the insulating sleeve 602, including the maximum and minimum iceball positions described above as well as intermediate iceball conditions between the maximum and minimum iceball positions.

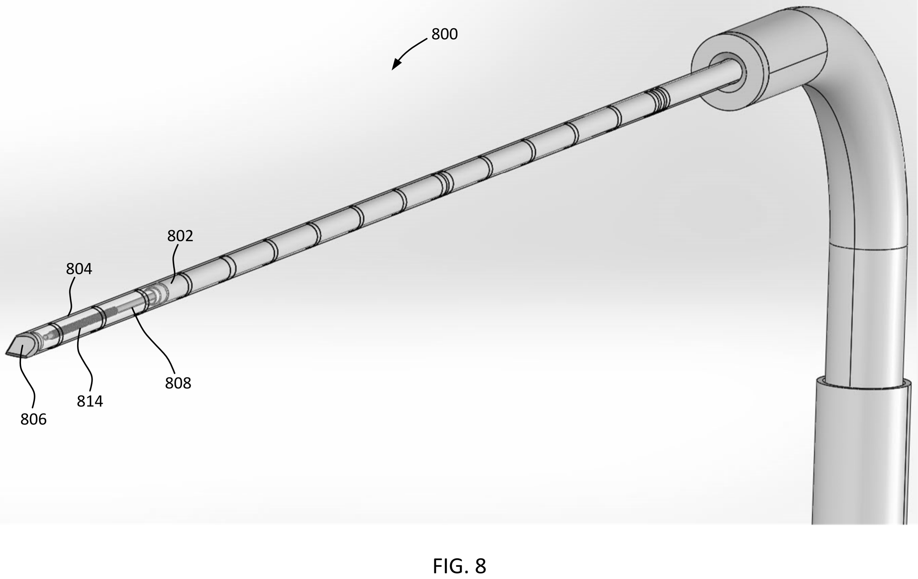

Turning now to FIG. 8, another example cryoprobe 800 is shown. In this example, the cryoprobe 800 may include a heater 814 that is positioned and/or configured differently from the configurations described above. The cryoprobe 800 is similar in that it includes an insulating sleeve 802, a needle 804, a tip 806, and a cryogen conduit 808. The heater 814, in this example, is positioned at or around the cryogen conduit 808. The heater 814 may be connected to the cryogen conduit 808 to remain in a fixed position in the needle 804. The heater 814 may be coiled or wrapped around the cryogen conduit 808. The outer diameter of the heater 814 is sized so that the insulating sleeve 802 may slide over the heater 814 when the insulating sleeve is adjusted to a desired axial position in the needle 804. In some positions, the heater 814 (or a portion of the heater 814) may be exposed or not covered by the insulating sleeve 802. When the insulating sleeve 802 is moved to position close to the tip 806, such as in a position to produce a minimum iceball, the insulating sleeve 802 may be radially outward of the heater 814 and cover the heater 814 or a portion thereof.

In this example, the heater 814 may be radially spaced away from the needle 804. The outer diameter of the heater 814 may be less than the inner diameter of the needle 804 to allow the insulating sleeve 802 to slide over the heater 814. In such a configuration, the heater 814 may transfer thermal energy to the needle 804 via convection when the heater 814 is energized. The heater 814 may be operated and controlled to achieve a desired temperature to perform a thaw, cautery, and/or track ablation procedure. The heater 814 may achieve temperatures of at least about 80 degrees Celsius.

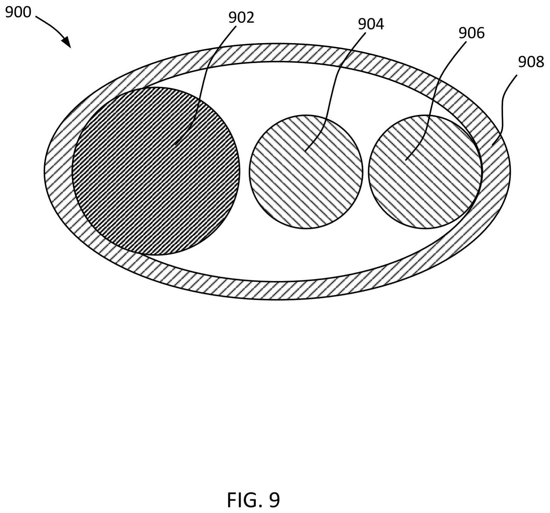

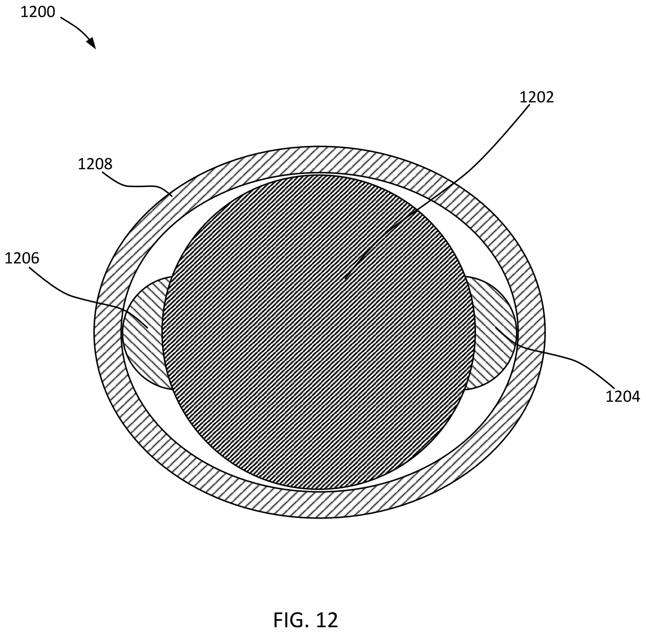

Referring now to FIGs. 9-14, various example multi-filar wire configurations are shown. The multi-filar heaters of the present disclosure, including the heaters described above, may be formed using one or more of the multi-filar wire configurations described. In one example configuration, a multi-filar wire 900 may include a structural member 902, a first heater wire 904, a second heater wire 906, and a cover 908. The structural member 902 may be a length of material such as a metal, alloy, or plastic material. In some examples, a steel, aluminum, or other material may be used. The structural member 902 may be used to provide rigidity to the multi-filar wire. The structural member 902 may also provide strength or support to the multi-filar wire 900 to prevent damage or breakage to the heater wires 904, 906. The structural member 902 may be the same size or have the same outer diameter as the heater wires 904, 906. In other examples, the structural member 902 may be larger or smaller or have a different outer diameter than the heater wires 904, 906. The structural member 902 may also allow the multi-filar wire 900 to be shaped in a desired configuration such as a coil, helix, or other shape as previously described.

In some examples, the structural member 902 may be deformable and may return to a desired shape. The structural member 902 may be formed of a spring metal and may be elongated and/or return to an original shape after the structural member 902 is deformed, elongate, or stretched by and external force. In other examples, the structural member 902 may be formed of a nickel titanium alloy or nitinol alloy. Such a material may allow the structural member to have a memory. When the memory material, such as nitinol, is elevated to a particular temperature, the structural member 902 may move or change to a desired shape at the predetermined temperature. For example, the structural member 902 may be used to move the multi-filar heater to a desired length or a desired coil shape without using an external force. The multi-filar heater may be heated and when a predetermined temperature is reached the memory of the material (such as nitinol) may move the heater to the desired length or desired shape.

The first heater member 904 and the second heater member 906 may have similar properties, sizes and shapes. In one example, the first heater member 904 and the second heater member 906 may be formed of a resistive metal material. In some examples, the first heater member 904 and the second heater member 906 may be made of Alloy 120 (Balco) material that may include about 70% nickel and about 30% iron. In other examples, the first heater member 904 and the second heater member 906 may be made of other resistive materials. The first heater member 904 and the second heater member 906 may heat and, in turn, heat the needle of the cryoprobe to a desired temperature, including cautery and track ablation temperatures. While not shown, the first heater member 904 and the second heater member 906 may include an insulating cover around the conductive portion of the member to electrically insulate the conductive portions from each other.

The first heater member 904 and the second heater member 906 may extend along a length of the heater from a handle of the cryoprobe toward and near a distal end of the needle. The first heater member 904 and the second heater member 906 may be joined to each at or near the distal end of the needle. The first heater member 904 and the second heater member 906 may form a heating circuit that heats when a current is passed through the heater members 904, 906. In this example, the first heater member 904 and the second heater member 906 are each positioned adjacent one another. The first heater member 904 and the second heater member 906 along with the structural member 902 are aligned or positioned next to each other in a linear relationship. In other examples, the first heater member 904 and the second heater member 906, and the structural member 902 may be oriented relative to each other in other configurations in other examples. In still other examples, the multi-filar wire 900 may include more than two heater members.

The multi-filar wire 900 also includes the cover 908. The cover 908 may surround and/or cover the structural member 902, first heater member 904, and the second heater member 906. The cover 908 may be made of an insulating material. The cover 908 may be a heat shrink or polymer tubing material. In some examples, the cover may be a thin layer of insulating material that is applied to the outer surface of the structural member and the heating wires. The cover 908 may join the wires or members to each other. In some examples, a polyimide material may be used for the cover 908. Other materials may be used in other examples.

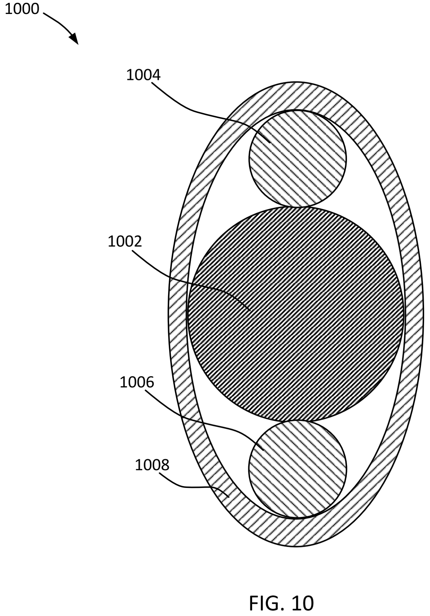

Turning now to FIG. 10, another example multi-filar wire 1000 is illustrated. In this example, the multi-filar wire 1000 may include a structural member 1002, a first heater member 1004, a second heater member 1006, and a cover 1008. Each of the structural member 1002, the first heater member 1004, the second heater member 1006, and the cover 1008 may be similar to the corresponding elements of multi-filar wire 900 previously described. In this example, the first heater member 1004 and the second heater member 1006 may be arranged differently relative to the structural member 1002. In this example, the first heater member 1004 is positioned on an opposite side of the structural member 1002 from the second heater member 1006.

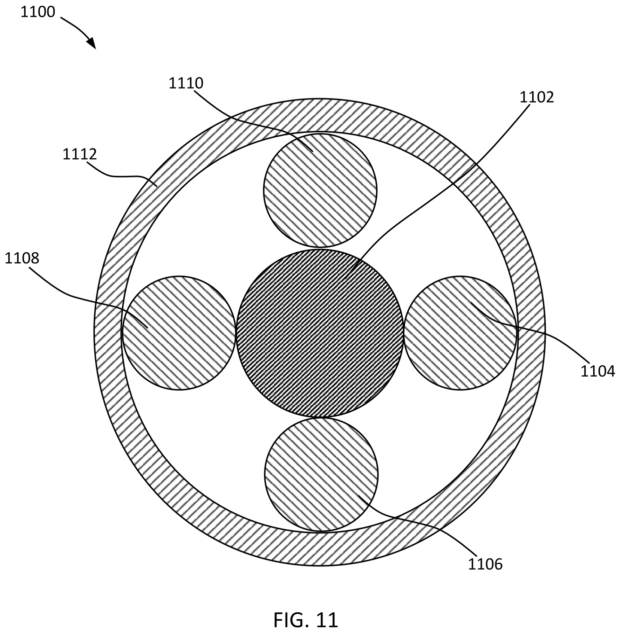

Turning now to FIG. 11, another example multi-filar wire 1100 is illustrated. In this example, the multi-filar wire 1100 may include a structural member 1102, a first heater member 1104, a second heater member 1106, a third heater member 1108, a fourth heater member 1110, and a cover 1112. Each of the structural member 1102, the first heater member 1104, the second heater member 1106, the third heater member 1108, the fourth heater member 1110, and the cover 1112 may be similar to the corresponding elements of multi-filar wire 900 previously described. In this example, the first heater member 1104, the second heater member 1106, the third heater member 1108, the fourth heater member 1110 may be positioned around a circumference of the structural member 1102. The first heater member 1104, the second heater member 1106, the third heater member 1108, and the fourth heater member 1110 may be equally spaced around the structural member 1102.

In another example, a multi-filar wire 1200 may include a first heater member 1204 and a second heater member 1206 that are deposited on a surface of the structural member 1202. The multi-filar wire 1200 may also include a cover 1208 similar to the covers previously described. The first heater member 1204 and the second heater member 1206 may be deposited by printing a conductive resistive ink on the structural member 1202. While only two heater members are shown in this example, other quantities and/or arrangements of the heater members may be used.

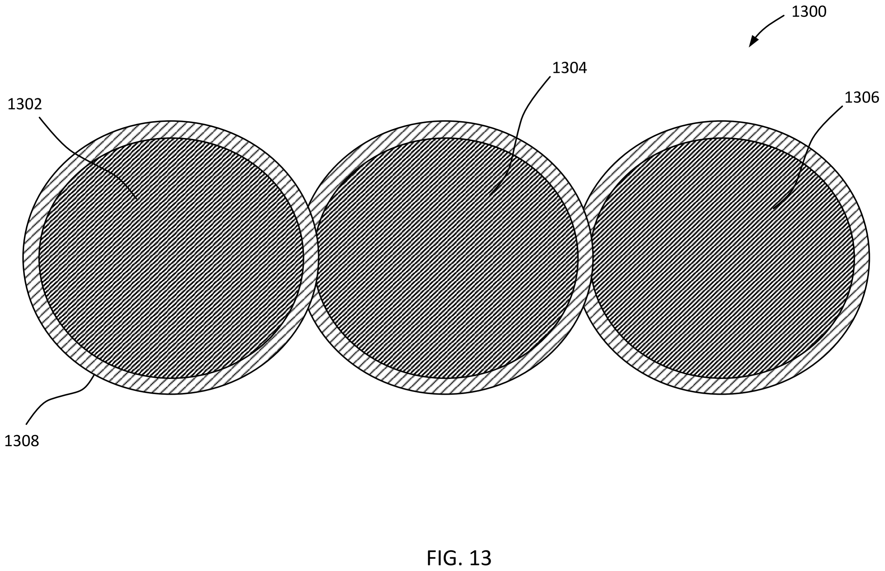

Turning now to FIG. 13, another example multi-filar wire 1300 is shown. In this example, the wire 1300 may be formed of three members including a first member 1302, a second member 1304, and a third member 1306. The first member 1302, the second member 1304, and the third member 1306 may have the same or a similar outer diameter. One of the first member 1302, the second member 1304, and the third member 1306 may be a structural member and may add rigidity and strength to the multi-filar wire 1300. Two of the other members of the first member 1302, the second member 1304, and the third member 1306 may be heating members that may be made of resistive heating material as previously described. The multi-filar wire 1300 may include a cover 1308 that may be applied to an outer surface of each of the first member 1302, the second member 1304, and the third member 1306 that may insulate them from one another and may join them together. Suitable materials such as those described above may be used for the cover 1308. In this example, three members are shown but in other examples, other numbers of members may be used.

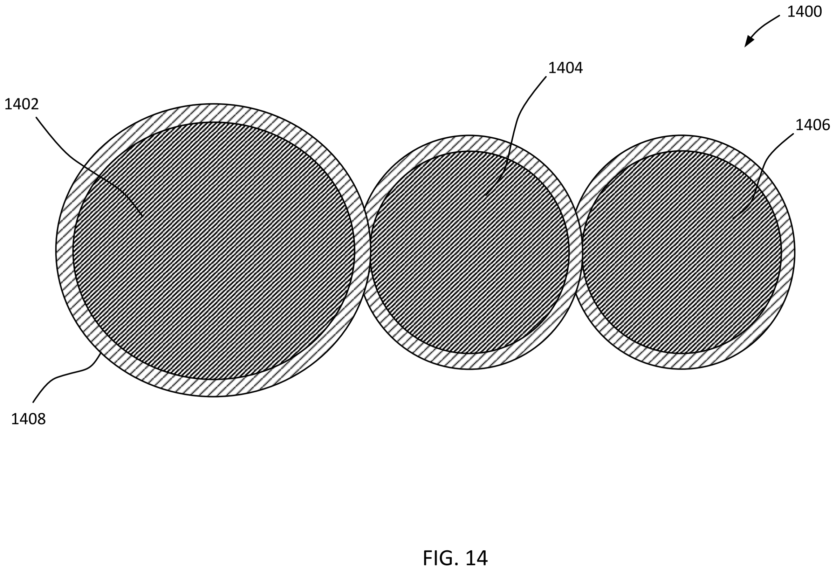

Turning now to FIG. 14, another example multi-filar wire 1400 is shown. The multi-filar wire 1400 may be similar to the multi-filar wire 1300 previously described. In this example, the multi-filar wire 1400 includes a structural member 1402, a first heater member 1404, and a second heater member 1406. In this example, the structural member 1402 may have a larger outer diameter than the first heater member 1404, and the second heater member 1406. The cover 1408 may be deposited on the outer surface of the structural member 1402, the first heater member 1404, and the second heater member 1406 to separate and join the members to each other. In the example, the structural member 1402, the first heater member 1404, and the second heater member 1406 may be positioned linearly such that a center of the structural member 1402, the first heater member, and the second heater member 1406 are linearly aligned. In other examples, other arrangements or orientations of the members relative to each other may be used.

While not shown in the examples, some embodiments may include one or more sensor wires. The sensor wires or members may be positioned in the cover and extend adjacent to the structural member and/or the heater members. In still other examples, the structural member and/or the heater members may be used to collect signal and/or information regarding operation of the cryoprobe, such as temperature information.

The following is a list of non-limiting illustrative embodiments disclosed herein:

Illustrative embodiment 1. A cryoprobe comprising: a needle defining an internal cavity; a cryogen conduit positioned in the internal cavity; and a multi-filar heater positioned radially outward of the cryogen conduit in the internal cavity configured to heat the needle to a predetermined temperature.

Illustrative embodiment 2. The cryoprobe of illustrative embodiment 1, further comprising an adjustable insulating sleeve positioned in the inner cavity radially outward of the cryogen conduit

Illustrative embodiment 3. The cryoprobe of illustrative embodiment 2, wherein the adjustable insulating sleeve is configured to move axially in the internal cavity of the needle.

Illustrative embodiment 4. The cryoprobe of any of illustrative embodiments 2 or 3, wherein the adjustable insulating sleeve is a vacuum sleeve.

Illustrative embodiment 5. The cryoprobe of any of illustrative embodiments 1 to 4, wherein the multi-filar heater comprises a coil of multi-filar wire.

Illustrative embodiment 6. The cryoprobe of any of illustrative embodiments 2 to 4, wherein the multi-filar heater is connected to the adjustable insulating sleeve and configured to extend or contract with a movement of the adjustable insulating sleeve.

Illustrative embodiment 7. The cryoprobe of any of illustrative embodiments 2 to 4 or 6, wherein a first end of the multi-filar heater is connected to the cryogen conduit and a second end of the heater is connected to the insulating sleeve.

Illustrative embodiment 8. The cryoprobe of any of illustrative embodiments 1 to 7, wherein the multi-filar heater comprises a multi-filar wire comprising a structural wire and a plurality of heater wires.

Illustrative embodiment 9. The cryoprobe of illustrative embodiment 8, wherein an outer diameter of the structural wire is greater than an outer diameter of each heater wire of the plurality of heater wires.

Illustrative embodiment 10. The cryoprobe of any of illustrative embodiments 8 or 9, wherein the multi-filar wire comprises a polyimide insulation cover.

Illustrative embodiment 11. The cryoprobe of any of illustrative embodiments 8 to 10, wherein the multi-filar wire comprises a helical shape.

Illustrative embodiment 12. The cryoprobe of any of illustrative embodiments 8 to 11, wherein the plurality of heater wires are deposited on an external surface of the structural wire.

Illustrative embodiment 13.The cryoprobe of any of illustrative embodiments 8 to 12, wherein each heater wire of the plurality of heater wires comprises an external insulating layer.

Illustrative embodiment 14. The cryoprobe of any of illustrative embodiments 8 to 13, wherein the structural wire comprises a nickel and titanium alloy configured to move to predetermined shape at a predetermined temperature.

Illustrative embodiment 15. The cryoprobe of any of illustrative embodiments 8 to 14, wherein the plurality of heater wires comprises a first heater wire and a second heater wire.

Illustrative embodiment 16. The cryoprobe of any of illustrative embodiments 8 to 15, wherein each heater wire of the plurality of heater wires are positioned adjacent to each other.

Illustrative embodiment 17. The cryoprobe of any of illustrative embodiments 8 to 15, wherein each heater wire of the plurality of heater wires are positioned around a circumference of the structural wire.

Illustrative embodiment 18. The cryoprobe of any of illustrative embodiments 1 to 17, wherein the multi-filar heater comprises at least one temperature sensing wire.

Illustrative embodiment 19. A cryoprobe comprising: a needle defining an internal cavity; a cryogen conduit positioned in the internal cavity; and a multi-filar heater positioned radially outward of the cryogen conduit in the internal cavity configured to heat the needle to a predetermined temperature, the multi-filar heater formed of at least one structural wire and a plurality of heater wires wherein at least two of the plurality of heater wires are connected at or near a distal end of the internal cavity to form a heating circuit.

Illustrative embodiment 20. The cryoprobe of illustrative embodiment 19, wherein the multi-filar heater comprises a helical shape contacting an inner surface of the needle.

Illustrative embodiment 21. A cryoablation apparatus comprising a cryo-controller, a cryogen delivery apparatus and the cryoprobe of any of the preceding illustrative embodiments.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A cryoprobe (112, 200, 300, 600, 800) comprising:
a needle (114, 204, 304, 604, 804) defining an internal cavity;
a cryogen conduit (208, 308, 608, 808) positioned in the internal cavity; and
a multi-filar heater (214, 314, 614, 814) positioned radially outward of the cryogen conduit in the internal cavity configured to heat the needle to a predetermined temperature, and optionally, the multi-filar heater comprises at least one temperature sensing wire.

2. The cryoprobe of claim 1, further comprising an adjustable insulating sleeve (202, 302, 602, 802) positioned in the inner cavity radially outward of the cryogen conduit (208, 308, 608, 808) wherein, optionally, the adjustable insulating sleeve (202, 302, 602, 802) is configured to move axially in the internal cavity of the needle (114, 204, 304, 604, 804), wherein, optionally, the adjustable insulating sleeve (202, 302, 602, 802) is a vacuum sleeve.

3. The cryoprobe of claim 1 or 2, wherein the multi-filar heater (214, 314, 614, 814) comprises a coil of multi-filar wire (900, 1000, 1100, 1200, 1300, 1400).

4. The cryoprobe of claim 2 or 3, wherein the multi-filar heater (214, 314, 614, 814) is connected to the adjustable insulating sleeve (202, 302, 602, 802) positioned and configured to extend or contract with a movement of the adjustable insulating sleeve.

5. The cryoprobe of claim 2, 3 or 4, wherein a first end (320) of the multi-filar heater (314) is connected to the cryogen conduit (308) and a second end (322) of the heater is connected to the insulating sleeve (302).

6. The cryoprobe of any one of the preceding claims, wherein the multi-filar heater comprises a multi-filar wire (900, 1000, 1100, 1200, 1300, 1400) comprising a structural wire (902, 1002, 1102, 1202, 1402) and a plurality of heater wires (904, 906, 1004, 1006, 1104, 1106, 1108, 1110, 1204, 1206, 1302, 1304, 1306, 1404, 1406), and optionally, the multi-filar wire comprises a polyimide insulation cover (908), and, optionally, the structural wire (902, 1002, 1102, 1202, 1402) comprises a nickel and titanium alloy configured to move to predetermined shape at a predetermined temperature.

7. The cryoprobe of claim 6, wherein an outer diameter of the structural wire (902, 1002, 1102, 1202, 1402) is greater than an outer diameter of each heater wire of the plurality of heater wires (904, 906, 1004, 1006, 1104, 1106, 1108, 1110, 1204, 1206, 1404, 1406).

8. The cryoprobe of claim 6 or 7, wherein the multi-filar wire comprises a helical shape.

9. The cryoprobe of claim 6, 7 or 8, wherein the plurality of heater wires (1204, 1206) are deposited on an external surface of the structural wire (1202).

10. The cryoprobe of any one of claims 6 to 9, wherein each heater wire of the plurality of heater wires (1302, 1304, 1306, 1404, 1406) comprises an external insulating layer (1308, 1408).

11. The cryoprobe of any one of claims 6 to 10, wherein the plurality of heater wires comprises a first heater wire (904) and a second heater wire (906).

12. The cryoprobe of any one of claims 6 to 11, wherein each heater wire (904, 906) of the plurality of heater wires are positioned adjacent to each other.

13. The cryoprobe of any one of claims 6 to 11, wherein each heater wire (1004, 1006, 1104, 1106, 1108, 1110, 1204, 1206) of the plurality of heater wires are positioned around a circumference of the structural wire.

14. A cryoprobe (112, 200, 300, 600, 800) comprising:
a needle (114, 204, 304, 604, 804) defining an internal cavity;
a cryogen conduit (208, 308, 608, 808) positioned in the internal cavity; and
a multi-filar heater (214, 314, 614, 814) positioned radially outward of the cryogen conduit in the internal cavity configured to heat the needle to a predetermined temperature, the multi-filar heater formed of at least one structural wire (902) and a plurality of heater wires (904, 906) wherein at least two of the plurality of heater wires (904, 906) are connected at or near a distal end of the internal cavity to form a heating circuit.

15. A cryoablation apparatus (100) comprising a cryo-controller (104), a cryogen delivery apparatus (106) and the cryoprobe (112) of claim 14.
